(19) Europäisches Patentamt
European Patent Office
Office européen des brevets

(11) Numéro de publication: **0 364 313 B1**

(12) **FASCICULE DE BREVET EUROPEEN**

(45) Date de publication de fascicule du brevet: **14.12.94**

(51) Int. Cl.[5]: **C07C 255/42**, C07C 255/37, C07D 455/04, G02F 1/35

(21) Numéro de dépôt: **89402474.4**

(22) Date de dépôt: **12.09.89**

Le dossier contient des informations techniques présentées postérieurement au dépôt de la demande et ne figurant pas dans le présent fascicule.

# (54) Composés organiques actif en optique non linéaire et dispositifs électrooptiques les contenant.

(30) Priorité: **15.09.88 FR 8812028**

(43) Date de publication de la demande:
**18.04.90 Bulletin 90/16**

(45) Mention de la délivrance du brevet:
**14.12.94 Bulletin 94/50**

(84) Etats contractants désignés:
**AT BE CH DE ES FR GB GR IT LI LU NL SE**

(56) Documents cités:
**EP-A- 0 244 051**
**DE-A- 2 345 189**

(73) Titulaire: **FLAMEL TECHNOLOGIES**
**Parc Club du Moulin à Vent**
**33, avenue du Docteur Georges Levy**
**F-69693 Venissieux Cédex (FR)**

(72) Inventeur: **Mignani, Gérard**
**2, avenue des Frères Lumière**
**F-69008 Lyon (FR)**
Inventeur: **Soula, Gérard**
**33, rue Nungesser**
**F-69330 Meyzieu (FR)**
Inventeur: **Meyrueix, Rémi**
**112, Cours Albert Thomas**
**F-69008 Lyon (FR)**

(74) Mandataire: **Ropital-Bonvarlet, Claude et al**
**Cabinet BEAU DE LOMENIE**
**51, avenue Jean-Jaurès**
**B.P. 7073**
**F-69301 Lyon Cédex 07 (FR)**

**EP 0 364 313 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne des composés organiques présentant une activité en optique non linéaire.

Elle se rapporte plus particulièrement à des composés organiques hyperpolarisables pouvant être ajoutés dans une matrice pour former un constituant d'un dispositif électrooptique.

Comme l'indiquent J. Zyss et I. Ledoux dans l'article paru dans "L'Echo des Recherches", 1er trimestre 1987 sous le titre "Molécules organiques et traitement du signal optique", le développement des télécommunications optiques requiert la réalisation de composants utilisant des matériaux de forte susceptibilité non linéaire du deuxième ou du troisième ordre.

De nombreux composés tant inorganiques qu'organiques sont utilisés sous différentes formes telles que solutions, polymères, polymères dopés, couches monomoléculaires, cristaux liquides, monocristaux, polymères cristaux liquides ou analogues.

Les composés organiques sont très intéressants car la synthèse d'une très grande variété de produits est généralement possible. De plus, la plupart des composés organiques présentent une résistance élevée aux agressions extérieures (humidité, acidité, oxydation, etc) et peuvent être ajoutés dans des matières telles que des films de polymère ou analogue.

On connaît par le document EP-A-244 051, des composés organiques constitués par des produits non centrosymétriques, comprenant un donneur d'électrons et un accepteur d'électrons reliés l'un à l'autre par une chaîne oléfinique linéaire contenant au moins trois paires de double et de simple liaisons conjuguées.

Ces produits n'ont pas une forte activité en optique non linéaire et sont, en outre, relativement instables, en raison des réactions d'isomérisation, dont ils peuvent être le siège.

J.F. Nicoud et R.J. Twieg dans leur mémoire intitulé "Design and synthesis of organic molecular compounds for efficient second harmonic generation" Ed. D.S. CHEMLA et J. ZYSS, 1987 répertorient différentes molécules susceptibles d'être actives en optique non linéaire.

Ces molécules ont comme squelette des chaînes carbonées comprenant généralement des cycles aromatiques substitués d'une part par des groupements donneurs d'électrons et d'autre part par des groupements accepteurs d'électrons.

La délocalisation des électrons engendre de fortes hyperpolarisabilités du troisième et second ordres lorsque la molécule est non-centrosymétrique.

Des efforts de recherche importants sont continuellement développés pour découvrir et fabriquer de nouveaux composés présentant une activité en optique non linéaire.

Ainsi, l'invention propose l'application à titre de composés organiques hyperpolarisables présentant une activité élevée en optique non linéaire, des produits ayant des formules générales suivantes :

(I)

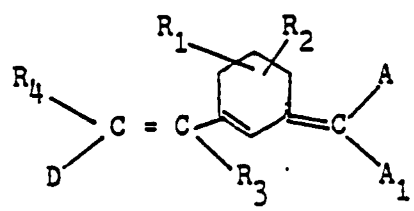

(II)

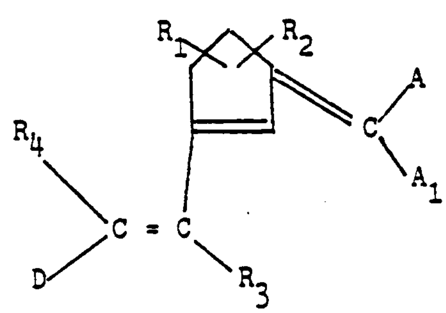

dans lesquelles : D est un groupement donneur d'électrons, A, $A_1$ identiques ou différents sont des groupements accepteurs d'électrons. $R_1$, $R_2$, $R_3$, $R_4$ sont des radicaux alkyles inférieurs comprenant de 1 à 3 atomes de carbone ou l'hydrogène.

Selon une autre caractéristique de l'invention, le groupement donneur d'électrons est un radical choisi dans le groupe comprenant les radicaux suivants :

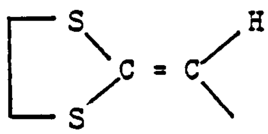
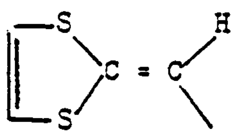
;
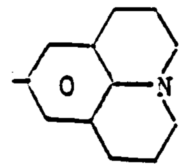

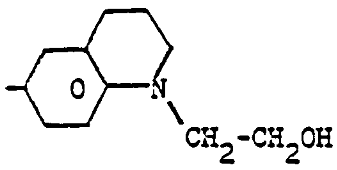
;
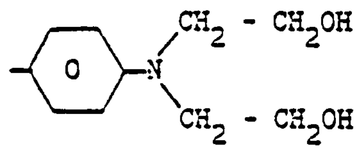
;
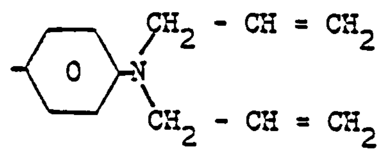

Le groupement D a, selon une autre caractéristique de l'invention la formule générale suivante :

$- R_6 - D_1$

Dans laquelle $R_6$ est un radical aryle, de préférence le radical benzylidène et $D_1$ est un radical donneur d'électrons choisi dans le groupe comprenant les radicaux amino, alkyamino, dialkylamino arylamino, hydroxyl, thiolo, alkylthio, arylthio, alkoxy, aryloxy, halogénoalkyl, oxy,

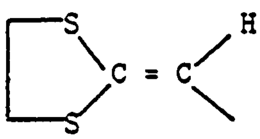
;
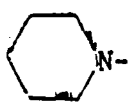
;
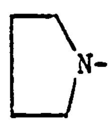
;
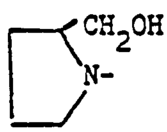
;
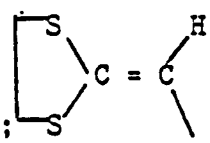

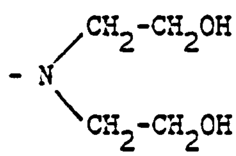
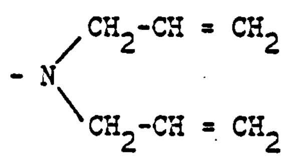

Le radical D préféré de l'invention est :

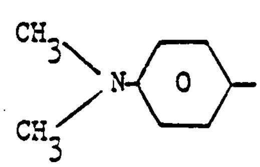

Les groupements A et $A_1$ qui peuvent être identiques ou différents représentent un radical accepteur d'électrons choisi dans le groupe comprenant les radicaux nitro, cyano, $-CO_2R_5$,$-PO_3(R_5)_2$, $R_5$ étant un radical alkyle inférieur comprenant de 1 à 3 atomes de carbone de préférence les radicaux éthyl ou propyl.

Les radicaux préférés de l'invention sont les radicaux cyano et nitro et plus particulièrement les associations cyano/cyano et cyano/nitro.

Selon une caractéristique de l'invention, les composés préférés sont les composés de configuration trans.

L'invention concerne également, à titre de produits nouveaux, les composés organiques ayant une activité élevée en optique non linéaire de formule générale II, cités ci-dessus.

Les composés de l'invention sont, par exemple, les composés de formules suivantes :

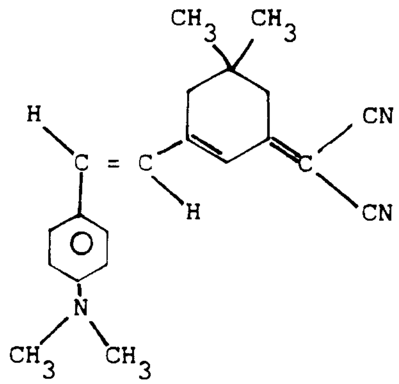

(A)

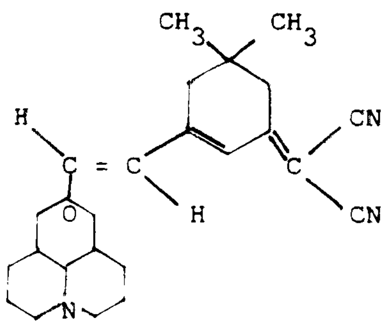

(B)

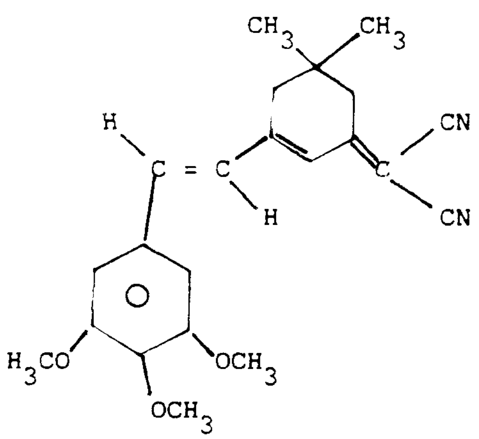

(C)

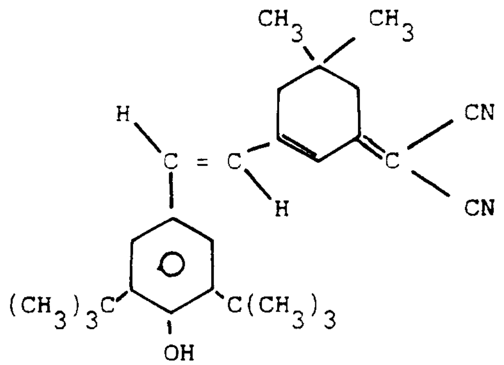

(D)

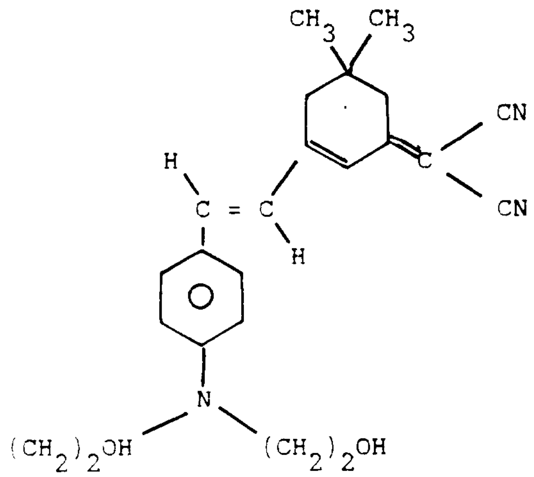

(E)

Ces composés peuvent être obtenus selon différents procédés de synthèse. Des exemples de procédés sont décrits dans les demandes de brevet allemand n° 2345189, l'article de Ralf LEMKE "Knoevenagel-Kondensationen in Dimethylformanid" publié dans Synthésis 5 (1974), 359 ou l'article "Solvatochromie von 80 µm in verschiedmen Alkokolen bei Arylidenisophorm - Abkömmlignen" du même auteur paru dans Chem. Ber, 103, (1970), 1894.

Brièvement, ce procédé consiste à mettre en oeuvre les réactions suivantes :

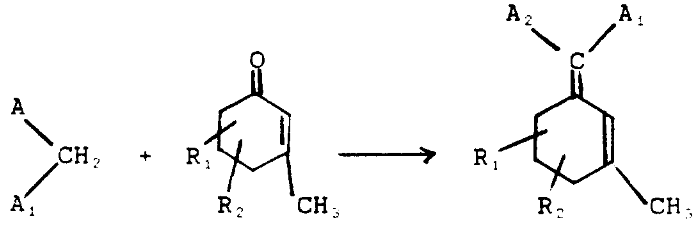

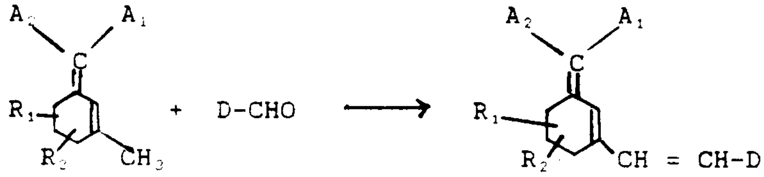

Cette dernière réaction est connue sous le nom de "Condensation de Knoevenagel".

Les composés de l'invention ont la propriété importante d'être actifs en optique non linéaire et donc sont susceptibles d'être utilisés dans les dispositifs de traitement électronique ou purement optique, notamment dans le domaine des transducteurs, des modulateurs, amplificateurs, etc...

En effet, l'activité des matériaux en optique non linéaire est déterminée par la valeur des coefficients $\beta$ et $\gamma$ d 'hyperpolarisabilité des second, troisième ou énième ordre.

L'hyperpolarisabilité d'un composé est directement reliée au moment dipolaire moléculaire par la relation fondamentale

$$\underline{\mu} = \underline{\mu o} + \alpha . \underline{E} + \beta \underline{E.E} + \gamma \underline{E.E.E} + ...$$

dans laquelle : $\mu$ et $\mu o$ représentent les moments dipolaires respectivement en présence et en absence de champ électromagnétique.

E représente le champ électrique ou électromagnétique local d'excitation.

$\alpha$ , $\beta$ , $\gamma$ représentent les coefficients de polarisabilité et d'hyperpolarisabilité.

En effet, le coefficient $\alpha$ est le coefficient de polarisabilité de la molécule et reflète son activité en optique linéaire.

Les coefficients $\beta$ et $\gamma$ représentent les coefficients d'hyperpolarisabilité respectivement de second et troisième ordre.

Ces coefficients reflètent l'anharmonicité du potentiel électrique dans la molécule et sont fortement dépendants de la symétrie et de la structure de celle-ci.

Par ailleurs, les coefficients d'ordre impair, tel que le coefficient $\gamma$ ne sont jamais nuls pour toutes molécules. Au contraire, les coefficients d'ordre pair tel que le coefficient $\beta$ sont nuls pour les molécules centrosymétriques.

Il est intéressant d'utiliser des molécules présentant un coefficient non nul pour les applications en optique non linéaire telles que, par exemple, les dispositifs électrooptiques, les modulateurs électrooptiques, les amplificateurs paramétriques, les dispositifs doubleur de fréquence.

Pour apprécier et mesurer le coefficient $\beta$ des molécules, on détermine celui-ci par comparaison avec celui d'une molécule de référence, à savoir, l'urée.

La mesure de l'hyperpolarisabilité moléculaire $\beta$ d'un composé peut être réalisée généralement dans une expérience de génération de deuxième harmonique. Elle se déroule en milieu solvant tel que, par exemple, l'acétone, l'eau, le diméthylsulfoxyde. La méthode connue sous le nom de méthode EFISH, est décrite dans les articles B.S. LEVINE et al Appl. Phys. Lett. vol 24 p. 445, 1974, et J.L. HOUDAR et al J. Chem. Phys. vol 67 p 1926, 1977.

Il est également possible de mesurer le produit $\mu$B, ($-\omega$; $\omega$, O) par la mesure de la susceptibilité électrooptique $\chi^{(2)}(-\omega; \omega, 0)$ d'un film dopé et polarisé de PMMA contenant N molécules actives par unité de volume. La mesure de $\chi^{(2)}(-\omega; \omega, 0)$ peut être réalisée par interférométrie comme cela est décrit dans l'article de K.D. SINGER et al J. Opt. Soc. Am. B vol 4 n°6 p. 968 et s. (1987). La relation entre $\mu\beta$ et $\chi^{(2)}$ est bien connue, on la trouvera par exemple dans l'article de K.D. SINGER et al. Appl. Phys. Lett. vol 49 n°5 p. 248 et s. (1986).

L'hyperpolarisibilité de la molécule peut également être exprimée par un coefficient $\beta\mu$ statique qui correspond à l'activité de la molécule à la fréquence nulle et donc donne une mesure de l'activité intrinsèque de la molécule.

Pour cela, on réalise une mesure de $\beta\mu$ à une fréquence donnée, par exemple selon l'une des méthodes décrites ci-dessus, puis on ramène cette valeur à une fréquence hypothétique nulle au moyen de modèles de calcul dits "modèle à deux niveaux".

La méthode de calcul du $\beta\mu$ statique est décrite dans l'article de K.D SINGER et al parue dans J-OPT.SOC.AM B/vol 4 n°6 pp. 968 et s (1987).

D'autres buts, détails et caractéristiques de l'invention apparaîtront plus clairement au vue des exemples donnés ci-dessous uniquement à titre indicatif et d'illustration.

Exemple 1 : fabrication du composé (A)

On introduit dans un ballon, 10 ml de pipéridine et 600 ml d'éthanol comme solvant puis 0,268 mole de

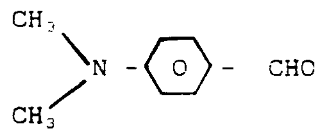

et 0,268 mole de

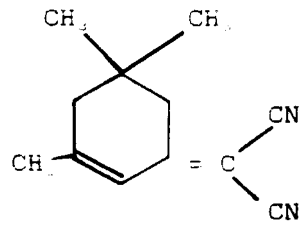

Le mélange est maintenu à reflux pendant 5 heures puis refroidi à température ambiante.

Le composé (A) est extrait par filtration et lavage à l'hexane.

Le produit recueilli est un solide rouge - violet présentant un point de fusion de 227 ° C.

Les analyses RMN, spectrographie de masse confirment la structure du composé A (voir page 4).

En outre, une analyse par spectrométrie ultra-violette en milieu chloroformique montre que la longueur d'onde (λ ) maximum d'absorption est de 504 nm.

Exemple 2 : fabrication du composé (B)

Dans 100 ml d'éthanol, on ajoute 0,268 mole de

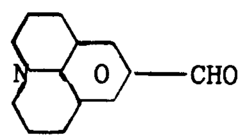

et

0,268 mole de

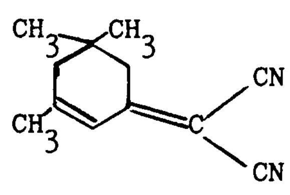

Le mélange est porté à reflux pendant 48 heures, puis le composé B est laissé cristallisé à température ambiante.

Après filtration et lavage à l'hexane, le composé (B) est récupéré sous la forme d'un solide violet présentant un point de fusion de 229 ° C.

Les analyses RMN, spectrographie infra-rouge et de masse confirme la structure du composé (B).

Ce composé présente en spectrométrie UV en milieu chloroformique, une longueur d'onde d'absorption maximale égale à 550 nm.

Exemple 3 : préparation du composé (C)

Selon un mode opératoire identique à celui de l'exemple 1 on fait réagir les composés

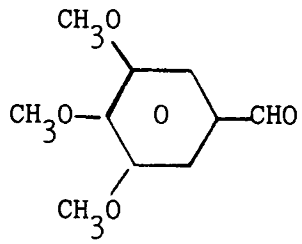 et 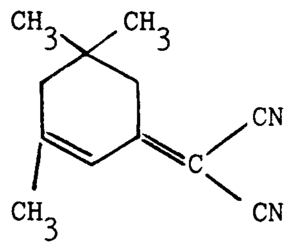

Après cristallisation, filtration et lavage à l'hexane, on récupére un solide orange présentant un point de fusion de 211,5 ° C.

Les analyses RMN, spectrographie IR et de masse confirme la structure du composé (C).

La longueur d'onde d'absorption maximale (λ max) en spectrométrie UV est de 420 nm ($CHCl_3$).

Exemple 4 : préparation du composé (D)

Selon le mode de réalisation décrit à l'exemple 2, on fait réagir les composés suivants :

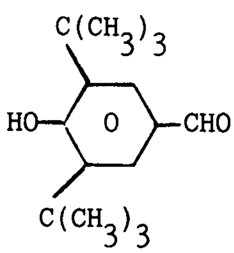 ; 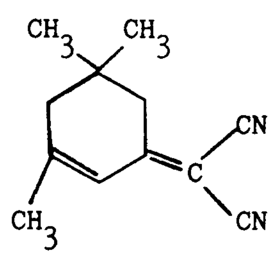

Après cristallisation, filtration et lavage à l'hexane puis recristallisation dans un mélange éthanol/acétone, on récupère un solide de couleur jaune orangé, présentant un point de fusion égal à 226 ° C et un λ max = 437 nm ($CHCl_3$).

De manière analogue aux exemples précédents les analyses confirment la structure du composé.

Exemple 5 : préparation du composé (E)

Ce produit est obtenu par réaction entre les composés suivants :

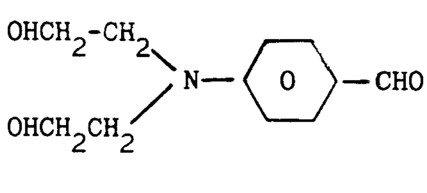 et 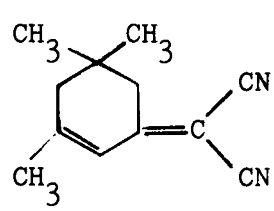

par mise en oeuvre du même mode opératoire que celui de l'exemple 2.

Le solvant est ensuite évaporé pour récupérer une huile rouge. Le composé (E) est extrait par séparation sur colonne chromatographique à base de gel de silice avec comme éluant l'acitate d'éthyle.

Le produit récupéré est un solide rouge-violet de point de fusion égal à 168 ° C et présentant un λ max = 494 nm ($CHCl_3$).

Comme pour les exemples précédents, les différentes analyses confirment la structure du composé E illustrée à la page 5.

Les résultats de la détermination du coefficient d'hyperpolarisabilité et du coefficient statique de ces différents produits sont rassemblés dans le tableau ci-dessous.

| Ex. | Composé | Coefficient d'hyperpolarisabilité $\beta\mu$ (-$\omega$ ;$\omega$ , o) à $\lambda$ = 633 nm | Coefficient statique $\beta\mu$ x $10^{-48}$ e.s.u. |
|---|---|---|---|
| 1 | A | 11710 x $10^{-48}$ esu | 2170 |
| 2 | B | 7390 x $10^{-48}$ esu | 750 |
| 3 | C | 880 x $10^{-48}$ esu | 330 |
| 4 | D | 2050 x $10^{-48}$ esu | 710 |
| 5 | E | 10610 x $10^{-48}$ esu | 1780 |

Les composés de l'invention sont utilisés dans des composants de dispositifs électrooptiques sous forme de matériaux, tel que par exemple sous forme de film, par incorporation dans une matrice, tel qu'un polymère, une résine etc. selon des techniques classiques et connues.

Ainsi, à titre d'exemple, les molécules préparées selon les exemples 1 à 5 sont incorporées dans un film de polymère transparent de 0,5 à 200 $\mu$m d'épaisseur, comme cela est décrit dans le brevet européen n° 218938. Comme polymères convenables, on peut citer, par exemple, le polyméthylméthacrylate, le polystyrène atactique.

Le film de polymère est chauffé à une température supérieure à sa température de transition vitreuse (Tg), puis soumis à un champ électrique intense pour orienter les molécules actives conformes à l'invention.

Le film est ensuite refroidi à une température inférieure à la température de transition vitreuse Tg pour ainsi geler la position orientée des molécules actives.

Un film contenant des molécules actives orientées de l'invention présente un coefficient électrooptique r et un coefficient de génération de second harmonique comparables à ceux des cristaux inorganiques habituellement utilisés dans ces applications tels que par exemple, le diphtalate de potassium, le diphtalate d'ammonium, le dihydrogénophtalate de potassium.

Le film présente, en outre, des avantages spécifiques tels que une basse constante diélectrique et une activité électrooptique d'origine essentiellement électronique.

Ces matériaux actifs en optoélectronique, notamment sous forme de film, sont susceptibles d'être utilisés dans des modulateurs électrooptiques, des guides actifs tels que des coupleurs directionnels, polariseurs, modulateurs intégrés etc...

**Revendications**

**1.** Application, à titre de composés organiques hyperpolarisables, présentant une activité en optique non linéaire, de produits présentant les formules générales suivantes :

(I)

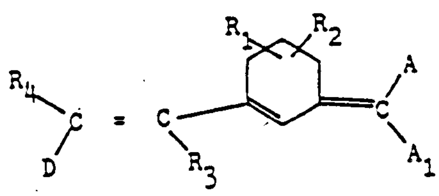

(II)

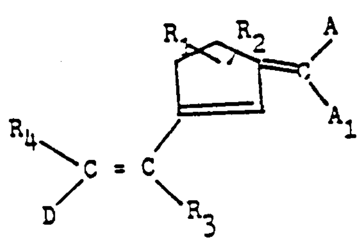

dans lesquelles :

- . D est un groupement donneur d'électrons,
- . A, $A_1$, identiques ou différents, sont des groupements accepteurs d'électrons,
- . $R_1$, $R_2$, $R_3$, $R_4$ sont des radicaux alkyles inférieurs comprenant de 1 à 3 atomes de carbone ou l'hydrogène.

**2.** Application selon la revendication 1, caractérisée en ce que le groupement D représente un radical donneur d'électrons choisi dans le groupe des radicaux suivants :

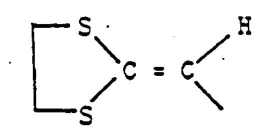 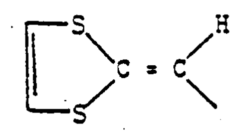 ; 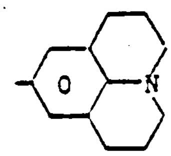

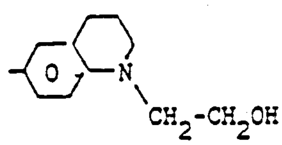 ; 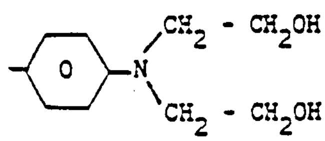 ; 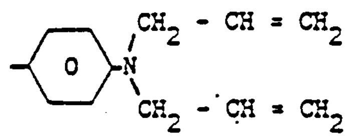

**3.** Application selon la revendication 1, caractérisée en ce que le groupement D donneur d'électrons a pour formule :

$- R_6 - D_1$

dans laquelle :

. $R_6$ est un radical aryle,

. $D_1$ est un radical choisi dans le groupe comprenant les radicaux amino, alkylamino, dialkylamino, arylamino, hydroxyl, thiolo, alkylthio, arylthio, aryloxy, halogénoalkyl, oxy,

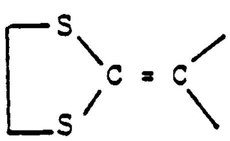 ; 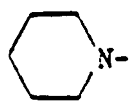 ; 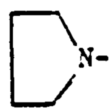 ; 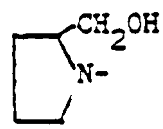 ; 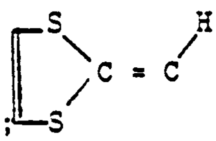

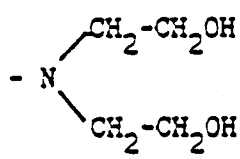 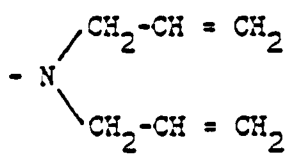

**4.** Application selon l'un quelconque des revendications 1 à 3, caractérisée en ce que les groupements A et A1, identiques ou différents, représentent un radical accepteur d'électrons choisi dans le groupe comprenant les radicaux nitro, cyano, $- CO_2R_5$, $- PO_3 (R_5)_2$, $R_5$ étant un radical alkyl inférieur, comprenant de 1 à 3 atomes de carbone.

**5.** Application selon la revendication 4, caractérisée en ce que A et $A_1$ représentent le radical cyano.

**6.** Application selon la revendication 4, caractérisée en ce que A et $A_1$ représentent, respectivement, un radical cyano et un radical nitro.

**7.** Application selon l'une quelconque des revendications 1 à 6 précédentes, caractérisée en ce que $D_1$ représente le radical diméthylamino.

**8.** Application selon l'une quelconque des revendications 1 à 7, caractérisée en ce que les composés ont une configuration trans.

9. Application selon l'une quelconque des revendications 1 à 8, caractérisée en ce que les composés ont les formules suivantes :

(A)

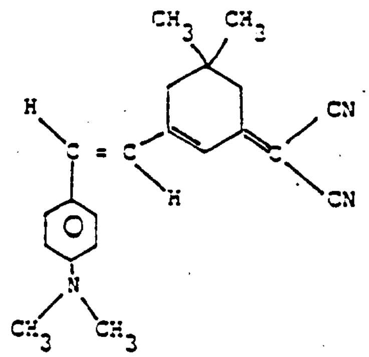

(B)

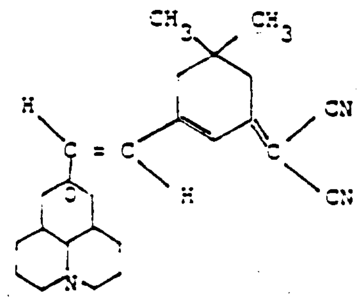

(C)

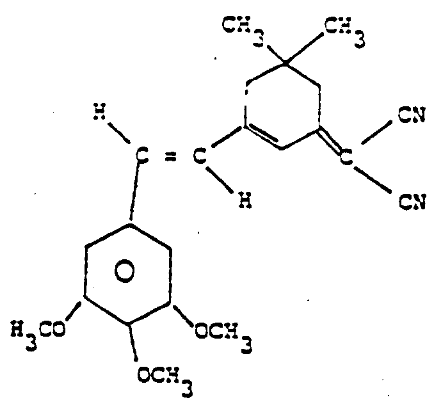

(D)

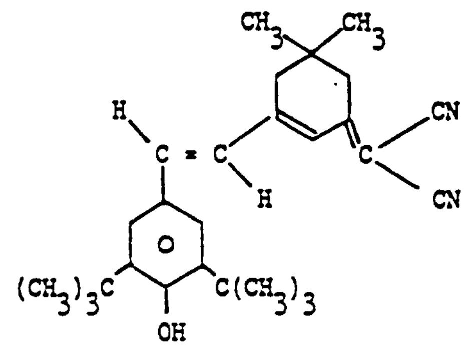

(E)

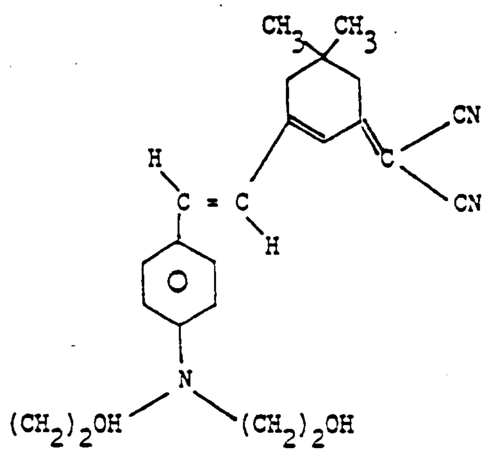

**10.** Composés organiques hyperpolarisables, présentant une activité en optique non linéaire, caractérisés en ce qu'ils sont constitués par les produits de formule générale (II) selon l'une quelconque des revendications 1 à 8.

**11.** Matériau actif en optique non linéaire, notamment susceptible de générer une harmonique du second ordre, caractérisé en ce qu'il comprend au moins un des composés mis en oeuvre dans l'application selon l'une des revendications 1 à 9 et/ou au moins un des composés selon la revendication 10.

**12.** Matériau selon la revendication 11, caractérisé en ce que le matériau est un film d'un polymère comprenant comme charge au moins un composé mis en oeuvre dans l'application selon l'une des revendications 1 à 9 et/ou au moins un des composés selon la revendication 10.

**13.** Dispositif optoélectrique comprenant un matériau actif en optique non linéaire selon la revendication 11 ou 12.

**Claims**

**1.** Application, as hyperpolarizable organic compounds, displaying a nonlinear optical activity, of products having the following general formulae:

(I) 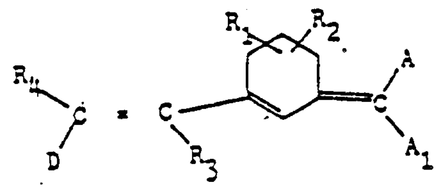

(II) 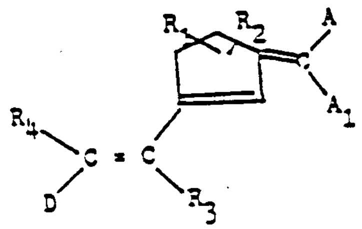

in which:

- . D is an electron donor group
- . A, $A_1$, which may be identical or different, are each an electron acceptor group,
- . $R_1$, $R_2$, $R_3$, $R_4$ are each lower alkyl radicals comprising from 1 to 3 carbon atoms or a hydrogen atom.

**2.** Application according to claim 1, characterized in that D is an electron donor radical selected from the group of the following radicals:

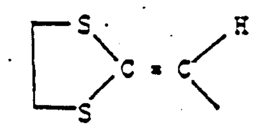 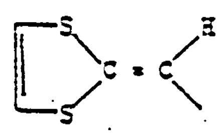 ; 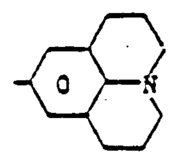

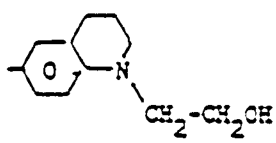 ; 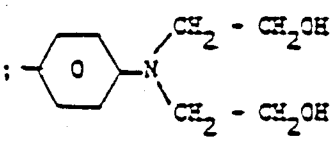 ; 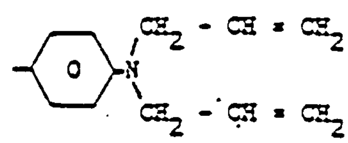

**3.** Application according to claim 1, characterized in that the electron donor group D has the formula:

$- R_6 - D_1$

in which:

- . $R_6$ is an aryl radical,
- . $D_1$ is a radical selected from the group comprising the amino, alkylamino, dialkylamino, arylamino, hydroxyl, thiolo, alkylthio, arylthio, aryloxy, halogenoalkyl, oxy radicals,

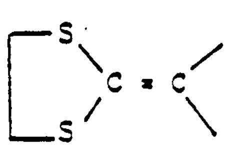 ; 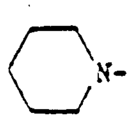 ; 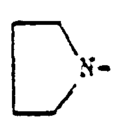 ; 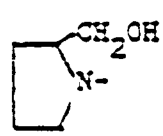 ; 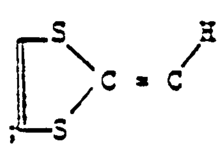

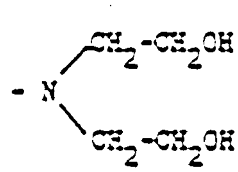 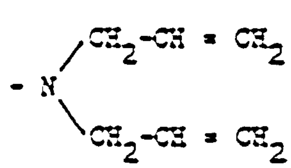

**4.** Application according to any one of claims 1 to 3, characterized in that the groups A and $A_1$, which may be identical or different, are each an electron acceptor radical selected from the group comprising the nitro, cyano, $-CO_2R_5$ or $-PO_3(R_5)_2$ radicals, $R_5$ being a lower alkyl radical, comprising from 1 to 3 carbon atoms.

**5.** Application according to claim 4, characterized in that A and $A_1$ are a cyano radical.

**6.** Application according to claim 4, characterized in that A and $A_1$ are a cyano radical and a nitro radical, respectively.

**7.** Application according to any one of preceding claims 1 to 6, characterized in that $D_1$ is the dimethylamino radical.

**8.** Application according to any one of clams 1 to 7, characterized in that the compounds have a trans-configuration.

**9.** Application according to any one of claims 1 to 8, characterized in that the compounds have the following formulae:

(A)

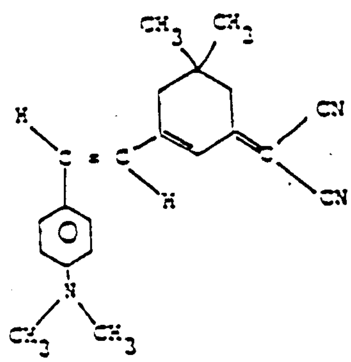

(B)

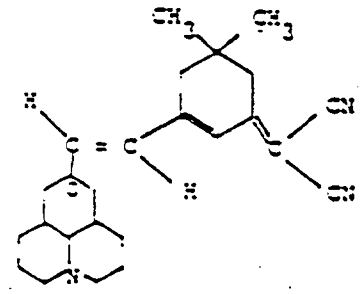

(C)

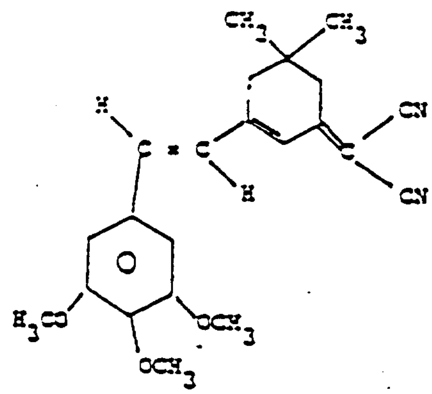

(D)

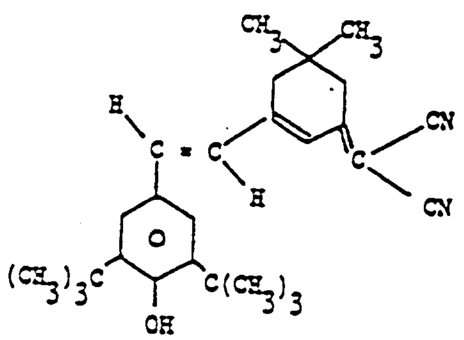

(E)

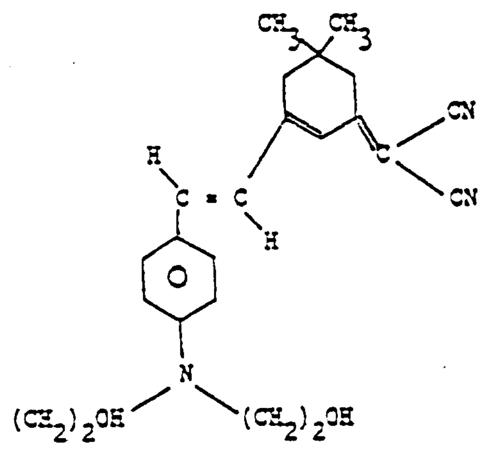

10. Hyperpolarisable organic compounds, having a nonlinear optical activity, characterized in that they are constituted by the products of general formula (II) according to any one of claims 1 to 8.

11. Nonlinearly optically active material, in particular capable of generating second order harmonics, characterized in that it comprises at least one of the compounds used in the application according to one of claims 1 to 9 and/or at least one of the compounds according to claim 10.

12. Material according to claim 11, characterized in that the material is a polymer film comprising as a filler at least one compound used in the application according to one of claims 1 to 9 and/or at least one of the compounds according to claim 10.

13. Optoelectrical device comprising a nonlinearly optically active material according to claim 11 or 12.

**Patentansprüche**

1. Verwendung von Verbindungen der folgenden allgemeinen Formeln

(I)

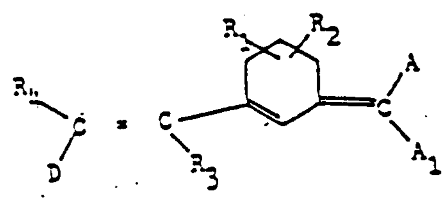

und

(II)

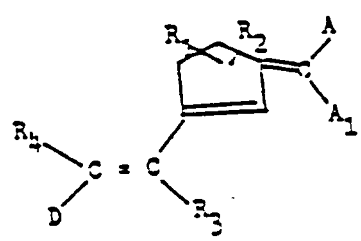

als hyperpolarisierbare organische Verbindungen, die nichtlineare optische Eigenschaften aufweisen, in denen bedeuten:

- D eine Elektronendonorgruppe,
- A und $A_1$ gleiche oder verschiedene Elektronenakzeptorgruppen,
- $R_1$, $R_2$, $R_3$, $R_4$ niedere Alkylgruppen mit 1 bis 3 Kohlenstoffatomen oder Wasserstoff.

2. Verwendung nach Anspruch 1, dadurch gekennzeichnet, daß die Gruppe D eine Elektronendonorgruppe darstellt, die unter folgenden Gruppen ausgewählt ist:

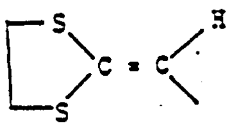 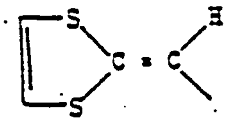 ; 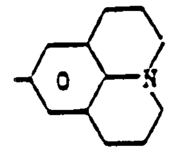

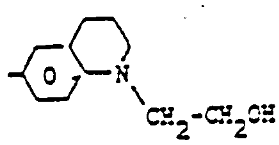 ; 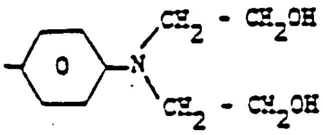 ; 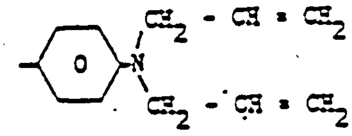

**3.** Verwendung nach Anspruch 1, dadurch gekennzeichnet, daß die Elektronendonorgruppe D die Formel

$- R_6 - D_1$

aufweist, worin bedeuten:

- $R_6$ eine Arylgruppe,
- $D_1$ eine Gruppe, die ausgewählt ist unter Amino, Alkylamino, Dialkylamino, Arylamino, Hydroxy, Thiol, Alkylthio, Arylthio, Aryloxy, Halogenalkyl, Oxy,

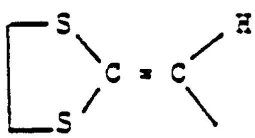 , 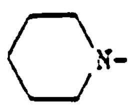 , 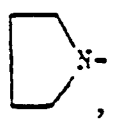 , 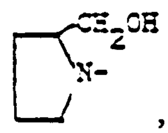 , 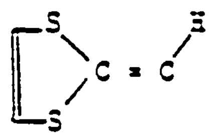

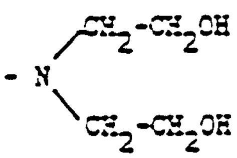 und 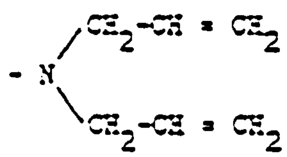 .

**4.** Verwendung nach einem der Ansprüche 1 bis 3, dadurch gekennzeichnet, daß die Gruppen A und $A_1$, die gleich oder verschieden sind, Elektronenakzeptorgruppen darstellen, die ausgewählt sind unter Nitro, Cyano, $-CO_2R_5$ und $PO_3(R_5)_2$, worin $R_5$ eine niedere Alkylgruppe ist, die 1 bis 3 Kohlenstoffatome aufweist.

**5.** Verwendung nach Anspruch 4, dadurch gekennzeichnet, daß A und $A_1$ Cyanogruppen darstellen.

**6.** Verwendung nach Anspruch 4, dadurch gekennzeichnet, daß A und $A_1$ eine Cyano- bzw. eine Nitrogruppe darstellen.

**7.** Verwendung nach einem der vorhergehenden Ansprüche 1 bis 6, dadurch gekennzeichnet, daß $D_1$ eine Dimethylaminogruppe darstellt.

**8.** Verwendung nach einem der Ansprüche 1 bis 7, dadurch gekennzeichnet, daß die Verbindungen in trans-Konfiguration vorliegen.

**9.** Verwendung nach einem der Ansprüche 1 bis 8, dadurch gekennzeichnet, daß die Verbindungen die folgenden Formeln aufweisen:

(A)

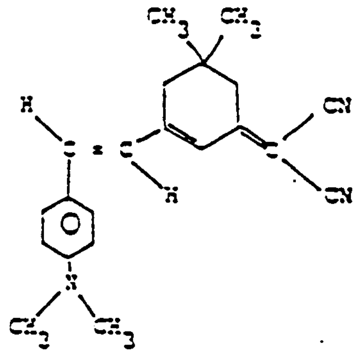

(B)

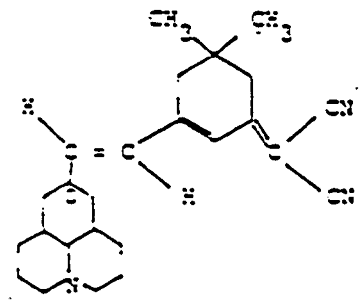

(C)

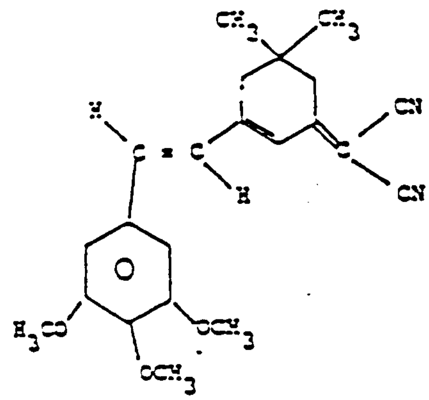

(D)

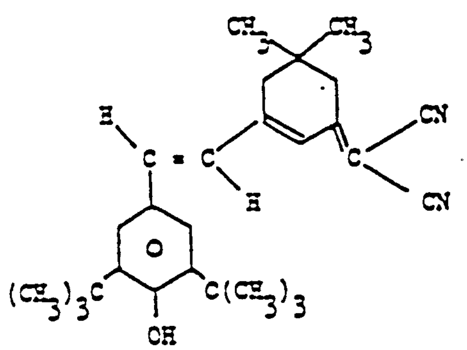

(E)

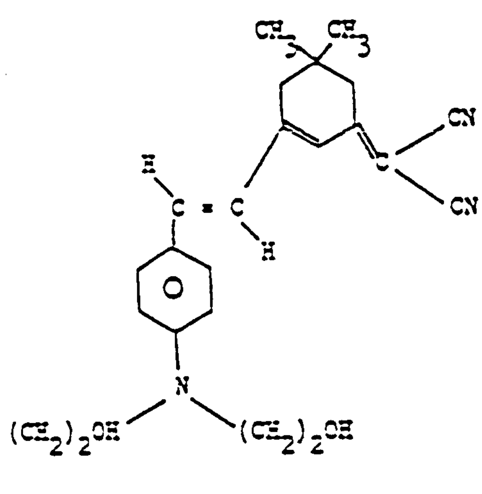

**10.** Hyperpolarisierbare organische Verbindungen, die nichtlineare optische Eigenschaften aufweisen, dadurch gekennzeichnet, daß sie aus den Verbindungen der allgemeinen Formel (II) nach einem der Ansprüche 1 bis 8 bestehen.

**11.** Material mit nichtlinearen optischen Eigenschaften, mit dem insbesondere die Oberschwingung zweiter Ordnung erzeugt werden kann, dadurch gekennzeichnet, daß es mindestens eine der bei der Verwendung nach einem der Ansprüche 1 bis 9 eingesetzten Verbindungen und/oder mindestens eine der

Verbindungen nach Anspruch 10 enthalt.

**12.** Material nach Anspruch 11, dadurch gekennzeichnet, daß es aus einem Polymerfilm besteht, der als Zusatz mindestens eine der bei der Verwendung nach einem der Ansprüche 1 bis 9 eingesetzten Verbindungen und/oder mindestens eine der Verbindungen nach Anspruch 10 enthält.

**13.** Elektrooptische Vorrichtung, die ein Material mit nichtlinearen optischen Eigenschaften nach Anspruch 11 oder 12 enthält.